# EUROPÄISCHE PATENTSCHRIFT

(11) **EP 3 209 387 B1**
(45) Veröffentlichungstag und Bekanntmachung des Hinweises auf die Patenterteilung: **24.04.2019**
(21) Anmeldenummer: 15780794.2
(22) Anmeldetag: 30.09.2015
(51) Int. Cl.: A61Q 15/00, A61K 8/73

(54) **VERWENDUNG VON CHITOSANEN IN SCHWEISSHEMMENDEN KOSMETISCHE MITTELN, WELCHE FREI SIND VON HALOGENIDEN UND/ODER HYDROXYHALOGENIDEN VON ALUMINIUM UND/ODER ZIRCONIU**
USE OF CHITOSANS IN ANTIPERSPIRANT COMPOSITIONS FREE OF HALOGEN OR HALOGENHYDROXYSALTS OF ALUMINUM AND OR ZIRCONIUM
UTILISATION DE CHITOSANE DANS COMPOSITIONS ANTITRANSPIRANTES QUI NE CONTIENNENT PAS LES HYDROCHLORIDES OU CHLORIDES D'ALUMINUM OU ET ZIRCONIUM

(30) Priorität: 24.10.2014 DE 102014221673
(43) Veröffentlichungstag der Anmeldung: 30.08.2017
(73) Patentinhaber: Henkel AG & Co. KGaA, 40589 Düsseldorf (DE)
(72) Erfinder: BANOWSKI, Bernhard, 40597 Düsseldorf (DE); EVERS, Stefan, 42781 Haan (DE); CLAAS, Marcus, 40723 Hilden (DE)
(86) Internationale Anmeldenummer: PCT/EP2015/072550
(87) Internationale Veröffentlichungsnummer: WO 2016/062507

(56) Entgegenhaltungen:
- EP-A1- 0 803 513
- WO-A2-96/23479

## Beschreibung

Die vorliegende Erfindung betrifft aluminiumsalzfreie und/oder aluminium-zirconiumsalzfreie schweißhemmende kosmetische Mittel, welche mindestens einen Stoff, ausgewählt aus der Gruppe von kosmetischen Ölen, welche bei 20 °C und 1.013 hPa flüssig sind, Riechstoffen und Wachsen, gegebenenfalls mindestens ein Treibmittel sowie mindestens ein spezielles Chitosan enthält. Der Zusatz des mindestens einen speziellen Chitosans resultiert in einer Beeinflussung der Schweißdrüse(n).

Weiterhin betrifft die vorliegende Erfindung eine Verpackungseinheit (Kit-of-parts) enthaltend ein kosmetisches Mittel mit mindestens einem schweißhemmenden Wirkstoff (M1) sowie ein erfindungsgemäßes kosmetisches Mittel (M2).

Darüber hinaus betrifft die vorliegende Erfindung die Verwendung von speziellen Chitosanen zur zumindest teilweisen Beeinflussung der Schweißdrüse(n).

Zudem betrifft die vorliegende Erfindung die Verwendung einer Kombination, welche mindestens einen Stoff, ausgewählt aus der Gruppe von kosmetischen Ölen, welche bei 20 °C und 1.013 hPa flüssig sind, Riechstoffen und Wachsen, gegebenenfalls mindestens ein Treibmittel sowie mindestens ein spezielles Chitosan enthält, zur Reduzierung und/oder Verhinderung von Schweiß, insbesondere Achselschweiß oder Schweiß anderer Körperregionen. Die Kombination gemäß der Erfindung enthält keine aluminiumhaltigen Verbindungen.

Schließlich betrifft die vorliegende Erfindung ein nicht-therapeutisches kosmetisches Verfahren zur Verhinderung und/oder Reduzierung der Transpiration des Körpers, bei welchem ein erfindungsgemäßes schweißhemmendes kosmetisches Mittel oder die Mittel (M1) und (M2) der erfindungsgemäßen Verpackungseinheit auf die Haut, insbesondere auf die Haut der Achselhöhlen, aufgetragen wird/werden und für mindestens 1 Stunde, vorzugsweise für mindestens 2 Stunden, bevorzugt für mindestens 4 Stunden, insbesondere für mindestens 6 Stunden, auf der Haut der Achselhöhlen verbleibt/verbleiben.

Das Waschen, Reinigen und Pflegen des eigenen Körpers stellt ein menschliches Grundbedürfnis dar und die moderne Industrie versucht fortlaufend, diesen Bedürfnissen des Menschen in vielfältiger Weise gerecht zu werden. Besonders wichtig für die tägliche Hygiene ist die anhaltende Beseitigung oder zumindest Reduzierung des Körpergeruchs und der Achselnässe. Im Stand der Technik sind zahlreiche spezielle deodorierende oder schweißhemmende Körperpflegemittel bekannt, welche für die Anwendung in Körperregionen mit einer hohen Dichte von Schweißdrüsen, insbesondere im Achselbereich, entwickelt wurden. Diese sind in den unterschiedlichsten Darreichungsformen konfektioniert, beispielsweise als Puder, in Stiftform, als Aerosolspray, Pumpspray, flüssige und gelförmige Roll-on-Applikation, Creme, Gel und als getränkte flexible Substrate (Deotücher).

Kosmetische Antitranspirantien des Standes der Technik enthalten neben mindestens einem Öl oder einem Wachs und einer Riechstoffkomponente bzw. einem Parfüm mindestens eine schweißhemmende Verbindung, insbesondere in Form von Halogeniden und/oder Hydroxyhalogeniden von Aluminium und/oder Zirconium. Diese schweißhemmenden Verbindungen verringern zum einen die Schweißsekretion des Körpers durch eine temporäre Verengung und/oder Verstopfung der Ausführungsgänge der Schweißdrüsen, so dass die Schweißmenge um etwa 20 bis 60 Prozent reduziert werden kann. Zum anderen weisen sie aufgrund ihrer antimikrobiellen Wirkung einen zusätzlichen desodorierenden Effekt auf.

Halogenide und/oder Hydroxyhalogenide von Aluminium und/oder Zirconium können in Verbindung mit dem sauren pH-Wert dieser Antitranspirantien bei einigen Anwendern zu unangenehmen Hautreaktionen führen. Darüber hinaus kann die Verwendung der vorgenannten schweißhemmenden Verbindungen zu einer Fleckenbildung auf der Kleidung führen.

Es besteht daher ein Bedarf schweißhemmende Halogenide und/oder Hydroxyhalogenide von Aluminium und/oder Zirconium gegen andere schweißhemmende kosmetische Wirkstoffe auszutauschen. Diese schweißhemmenden Wirkstoffe sollen eine gute schweißhemmende Wirkung, eine gute Hautverträglichkeit sowie eine einfache Formulierbarkeit aufweisen. Darüber hinaus sollen diese schweißhemmenden Wirkstoffe keinen negativen Einfluss auf die Lagerstabilität der schweißhemmenden kosmetischen Mittel aufweisen.

Der vorliegenden Erfindung lag die Aufgabe zugrunde, ein schweißhemmendes kosmetisches Mittel bereitzustellen, welches die Nachteile des Standes der Technik vermeidet bzw. zumindest abschwächt und welches eine gute Hautverträglichkeit bei gleichzeitig zuverlässiger Verminderung der Achselnässe besitzt. Darüber hinaus soll das schweißhemmende kosmetische Mittel eine hohe Lagerstabilität aufweisen.

Es wurde nun überraschend gefunden, dass der Einsatz mindestens eines speziellen Chitosans in kosmetischen Mitteln ohne aluminiumhaltige Verbindungen, insbesondere ohne schweißhemmende Halogenide und/oder Hydroxyhalogenide von Aluminium und/oder Zirconium, in einer schweißhemmenden Wirkung resultiert, welche mit der schweißhemmenden Wirkung von Formulierungen mit Aluminiumsalzen und/oder Aluminium-Zirkonium-Komplexen nahezu vergleichbar ist.

Gegenstand der vorliegenden Erfindung ist somit ein schweißhemmendes kosmetisches Mittel, enthaltend
a) mindestens einen Stoff, ausgewählt aus der Gruppe von kosmetischen Ölen, welche bei 20 °C und 1.013 hPa flüssig sind, Riechstoffen und Wachsen,
b) Treibmittel in einer Gesamtmenge von 0 bis 99 Gew.-%, bezogen auf das Gesamtgewicht des schweißhemmenden kosmetischen Mittels, und
c) mindestens ein Chitosan mit einer Viskosität von 15 bis 15.000 mPa*s, wobei das Chitosan die Formel (I) aufweist 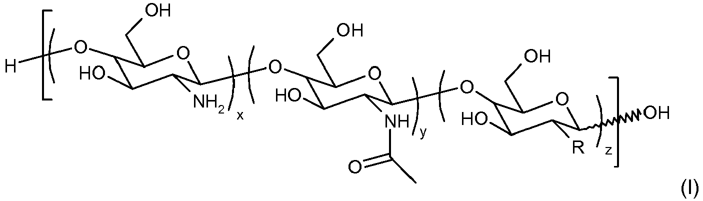 worin
   x und z, jeweils unabhängig voneinander, für ganze Zahlen von 5 bis 25.000 stehen,
   y für ganze Zahlen von 1 bis 25.000 steht,
   R für eine NH₂-Gruppe oder *-NH₃⁺CH₃CH(OH)C(O)O⁻ oder *-NH₃⁺OHCH₂C(O)O⁻ steht,
   mit der Maßgabe, dass die Reihenfolge der in der eckigen Klammer enthaltenden Einheiten mit den Indices x, y und z beliebig ist und dass der Rest R über * gebunden ist, und
d) keine aluminiumhaltigen Verbindungen.

Durch den Einsatz des mindestens einen Chitosans in den erfindungsgemäßen schweißhemmenden kosmetischen Mitteln erfolgt - ohne sich auf diese Theorie beschränken zu wollen - eine gezielte Beeinflussung der Schweißdrüse(n). Diese gezielte Beeinflussung der Schweißdrüse(n) kann beispielsweise in einer Ausfällung des mindestens einen Chitosans bei pH-Werten, welche ausschließlich innerhalb der Ausführungsgänge der Schweißdrüsen vorliegen, bestehen. Auf diese Art und Weise kann eine effektive Verstopfung der Ausführungsgänge der Schweißdrüsen gewährleistet werden, ohne dass die schweißhemmende Wirkung des erfindungsgemäßen kosmetischen Mittels durch vorzeitige unerwünschte Ausfällung aufgrund des Zusatzes des mindestens einen speziellen Chitosans vermindert wird. Weiterhin kann die gezielte Beeinflussung der Schweißdrüse(n) jedoch auch in einer Störung des Ladungsgleichgewichts innerhalb der Schweißdrüse(n) bestehen, welche zu einer Beeinflussung der Schweißproduktion, insbesondere zu einer Verminderung der Schweißproduktion, führt. Somit wird selbst bei Abwesenheit aluminiumhaltiger Verbindungen, insbesondere schweißhemmender Halogenide und/oder Hydroxyhalogenide von Aluminium und/oder Zirconium, eine effektive Verminderung des Achselschweißes gewährleistet.

Unter dem Begriff "schweißhemmend" wird erfindungsgemäß die Verminderung bzw. Reduzierung der Transpiration der Schweißdrüsen des Körpers verstanden.

Weiterhin wird unter dem Begriff "kosmetisches Öl" im Sinne der vorliegenden Erfindung ein für die kosmetische Verwendung geeignetes Öl verstanden, welches mit Wasser nicht in allen Mengen mischbar ist. Bei dem erfindungsgemäß verwendeten kosmetischen Öl handelt es sich weder um Riechstoffe, noch um ätherische Öle.

Zudem werden unter dem Begriff "Riechstoffe" im Sinne der vorliegenden Erfindung Substanzen mit einer Molmasse von 74 bis 300 g/mol verstanden, welche mindestens eine osmophore Gruppe im Molekül enthalten und einen Geruch und/oder Geschmack aufweisen, d. h. sie sind in der Lage, die Rezeptoren der Haarzellen des olfaktorischen Systems zu erregen. Osmophore Gruppen sind kovalent an das Molekülgerüst gebundene Gruppen in Form von Hydroxygruppen, Formylgruppen, Oxogruppen, Alkoxycarbonylgruppen, Nitrilgruppen, Nitrogruppen, Azidgruppen etc. In diesem Zusammenhang fallen unter den Begriff "Riechstoffe" im Sinne der vorliegenden Erfindung auch bei 20 °C und 1.013 hPa flüssige Parfümöle, Parfüme, oder Parfümölbestandteile.

Darüber hinaus werden unter dem Begriff "Wachse" im Rahmen der vorliegenden Erfindung Substanzen verstanden, welche bei 20 °C knetbar oder fest bis brüchig hart sind, eine grobe bis feinkristalline Struktur aufweisen und farblich durchscheinend bis opak, aber nicht glasartig sind. Weiterhin schmelzen diese Substanzen über 25 °C ohne Zersetzung, sind wenig oberhalb des Schmelzpunktes leicht flüssig (wenig viskos), weisen eine stark temperaturabhängige Konsistenz und Löslichkeit auf und sind unter leichtem Druck polierbar.

Weiterhin werden unter dem Begriff "Chitosane" erfindungsgemäß Deacetylierungsprodukte des Chitins verstanden, welche einen Deacetylierungsgrad von mehr als 40 % aufweisen und welche in 1%igen wässrigen Lösungen geeigneter Säuren, beispielsweise Essigsäure, Ameisensäure, Citronensäure, Milchsäure oder Salzsäure, löslich sind. Die Viskosität der erfindungsgemäß eingesetzten Chitosane wird unter Verwendung einer 1 Gew.-%igen Lösung von Chitosan, bezogen auf das Gesamtgewicht der Lösung, in 1 %iger Essigsäure unter Verwendung eines Brookfield RVDV II+, Spindel Nr. 2, 20 rpm bei 20 °C bestimmt.

Weiterhin werden unter dem Begriff "aluminiumhaltige Verbindungen" im Rahmen der vorliegenden Erfindung insbesondere Chloride, Bromide und lodide des Aluminiums und des Zirconiums sowie Verbindungen der Formeln Al(OH)_{y}X und Zr(OH)_{z}X verstanden, wobei X in den vorgenannten Formeln für ein Halogenidion steht.

Zudem sind unter dem Begriff der "Fettsäuren", wie er im Rahmen der vorliegenden Erfindung verwendet wird, aliphatische Carbonsäuren zu verstehen, welche unverzweigte oder verzweigte Kohlenstoffreste mit 4 bis 40 Kohlenstoffatomen aufweisen. Die im Rahmen der vorliegenden Erfindung eingesetzten Fettsäuren können sowohl natürlich vorkommende als auch synthetisch hergestellte Fettsäuren sein. Weiterhin können die Fettsäuren einfach oder mehrfach ungesättigt sein.

Schließlich sind unter dem Begriff der "Fettalkohole" im Rahmen der vorliegenden Erfindung aliphatische, einwertige, primäre Alkohole zu verstehen, welche unverzweigte oder verzweigte Kohlenwasserstoffreste mit 4 bis 40 Kohlenstoffatomen aufweisen. Die im Rahmen der Erfindung eingesetzten Fettalkohole können auch ein- oder mehrfach ungesättigt sein.

Die Angabe Gew.-% bezieht sich vorliegend, sofern nichts anderes angegeben ist, auf das Gesamtgewicht der treibmittelfreien erfindungsgemäßen schweißhemmenden kosmetischen Mittel.

Als ersten Bestandteil a) enthalten die erfindungsgemäßen kosmetischen Mittel mindestens einen Stoff, welcher aus der Gruppe von kosmetischen Ölen, welche bei 20 °C und 1.013 hPa flüssig sind, Riechstoffen und Wachsen ausgewählt ist.

Im Rahmen der vorliegenden Erfindung ist das bei 20°C und 1.013 hPa flüssige kosmetische Öl ausgewählt aus der Gruppe von (i) flüchtigen cyclischen Siliconölen, insbesondere cylischen und linearen Siliconölen; (ii) flüchtigen Nichtsiliconölen, insbesondere flüssigen Paraffinölen und Isoparaffinölen; (iii) nichtflüchtigen Siliconölen; (iv) nichtflüchtigen Nichtsiliconölen; sowie (v) deren Mischungen.

Der Begriff "flüchtiges Öl" bezeichnet erfindungsgemäß Öle, welche bei 20 °C und einem Umgebungsdruck von 1.013 hPa einen Dampfdruck von 2,66 Pa bis 40.000 Pa (0,02 bis 300 mm Hg), vorzugsweise von 10 bis 12.000 Pa (0,1 bis 90 mm Hg), weiter bevorzugt von 13 bis 3.000 Pa (0,1 bis 23 mm Hg), insbesondere von 15 bis 500 Pa (0,1 bis 4 mm Hg), aufweisen.

Darüber hinaus werden unter dem Begriff "nichtflüchtige Öle" im Sinne der vorliegenden Erfindung Öle verstanden, welche bei 20 °C und einem Umgebungsdruck von 1.013 hPa einen Dampfdruck von weniger als 2,66 Pa (0,02 mm Hg) aufweisen.

Es kann erfindungsgemäß bevorzugt sein, Mischungen von flüchtigen Siliconölen und flüchtigen Nichtsiliconölen in den erfindungsgemäßen schweißhemmenden kosmetischen Mitteln einzusetzen, da hierdurch ein trockeneres Hautgefühl erreicht wird. Weiterhin kann es im Rahmen der vorliegenden Erfindung bevorzugt sein, wenn die schweißhemmenden kosmetischen Mittel ein nichtflüchtiges Siliconöl und/oder ein nichtflüchtiges Nichtsiliconöl enthalten, um unlösliche Bestandteile, wie Talkum oder auf der Haut angetrocknete Inhaltsstoffe, zu maskieren.

Erfindungsgemäß besonders bevorzugt ist der Einsatz von Mischungen von nichtflüchtigen und flüchtigen kosmetischen Ölen, da auf diese Weise Parameter wie Hautgefühl, Sichtbarkeit des Rückstands und Stabilität des erfindungsgemäßen schweißhemmenden kosmetischen Mittels eingestellt und das Mittel somit besser an die Bedürfnisse der Verbraucher angepasst werden kann.

Die im Rahmen der vorliegenden Erfindung einsetzbaren flüchtigen und nichtflüchtigen Siliconöle sowie flüchtigen und nichtflüchtigen Nichtsiliconöle sind beispielsweise in den Offenlegungsschriften DE 10 2010 063 250 A1 und DE 10 2012 222 692 A1 offenbart.

Gemäß einer bevorzugten Ausführungsform der vorliegenden Erfindung ist das bei 20 °C und 1.013 hPa flüssige kosmetische Öl in einer Gesamtmenge von 0,02 bis 98 Gew.-%, vorzugsweise von 2 bis 85 Gew.-%, bevorzugt von 4 bis 75 Gew.-%, weiter bevorzugt von 6 bis 70 Gew.-%, noch weiter bevorzugt von 8 bis 60 Gew.-%, insbesondere von 8 bis 20 Gew.-%, bezogen auf das Gesamtgewicht des treibmittelfreien schweißhemmenden kosmetischen Mittels, enthalten.

Als Bestandteil a) der erfindungsgemäßen kosmetischen Mittel kann ebenfalls mindestens ein Riechstoff enthalten sein. Bevorzugt werden jedoch Mischungen verschiedener Riechstoffe verwendet, welche gemeinsam eine ansprechende Duftnote erzeugen. Im Rahmen der vorliegenden Erfindung einsetzbare Riechstoffe sind beispielsweise in der Offenlegungsschrift DE 10 2010 063 250 A1 offenbart.

Besonders ansprechend riechende erfindungsgemäße schweißhemmende kosmetische Mittel werden erhalten, wenn der mindestens eine Riechstoff in einer Gesamtmenge von 0,00001 bis 15 Gew.-%, vorzugsweise von 0,001 bis 9 Gew.-%, bevorzugt von 0,01 bis 8 Gew.-%, weiter bevorzugt von 0,1 bis 7 Gew.-%, noch weiter bevorzugt von 0,2 bis 6 Gew.-%, insbesondere von 0,2 bis 2 Gew.-%, bezogen auf das Gesamtgewicht des treibmittelfreien schweißhemmenden kosmetischen Mittels, enthalten ist.

Weiterhin können die erfindungsgemäßen schweißhemmenden kosmetischen Mittel als Bestandteil a) ein Wachs enthalten. Bevorzugt ist dieses Wachs ausgewählt aus der Gruppe von (i) Fettsäureglycerinmono-, -di- und -triestern; (ii) Butyrospermum Parkii (Shea Butter); (iii) Estern von gesättigten, einwertigen C₈₋₁₈-Alkoholen mit gesättigten C₁₂₋₁₈-Monocarbonsäuren; (iv) linearen, primären C_{12-C24}-Alkanolen; (v) Estern aus einem gesättigten, einwertigen C₁₆₋₆₀-Alkanol und einer gesättigten C_{8-C36}-Monocarbonsäure; (vi) Glycerintriestern von gesättigten linearen C₁₂₋₃₀-Carbonsäuren, die hydroxyliert sein können; (vii) natürlichen pflanzlichen Wachsen; (viii) tierischen Wachsen; (ix) synthetischen Wachsen; sowie (x) deren Mischungen. Im Rahmen der vorliegenden Erfindung bevorzugt einsetzbare Wachse sind in der Offenlegungsschrift DE 10 2012 222 692 A1 offenbart.

Im Rahmen der vorliegenden Erfindung ist es bevorzugt, wenn das Wachs in einer Gesamtmenge von 0,01 bis 50 Gew.-%, vorzugsweise von 3 bis 40 Gew.-%, bevorzugt von 5 bis 30 Gew-%, insbesondere von 6 bis 25 Gew.-%, bezogen auf das Gesamtgewicht des treibmittelfreien schweißhemmenden kosmetischen Mittels, enthalten ist.

Gemäß einer Ausführungsform der vorliegenden Erfindung kann es vorgesehen sein, dass die erfindungsgemäßen schweißhemmenden kosmetischen Mittel als Bestandteil b) ein Treibmittel in einer Gesamtmenge von 0 bis 99 Gew.-%, bezogen auf das Gesamtgewicht des treibmittelfreien schweißhemmenden kosmetischen Mittels, enthalten. Wenn die erfindungsgemäßen kosmetischen Mittel ein Treibmittel enthalten, ist dieses bevorzugt in einer Gesamtmenge von 1 bis 98 Gew.-%, vorzugsweise von 20 bis 90 Gew.-%, bevorzugt von 30 bis 85 Gew-%, insbesondere von 40 bis 75 Gew.-%, bezogen auf das Gesamtgewicht des schweißhemmenden kosmetischen Mittels, enthalten. In diesem Fall sind die erfindungsgemäßen kosmetischen Mittel als treibgasgetriebene Aerosole konfektioniert. Bevorzugte Treibmittel (Treibgase) sind Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, Tetrafluoropropene und zwar sowohl einzeln als auch in deren Mischungen. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann.

Als dritten Bestandteil c) enthält das erfindungsgemäße schweißhemmende kosmetische Mittel mindestens ein spezielles Chitosan gemäß der Formel (I).

Erfindungsgemäße kosmetische Mittel enthalten mindestens ein Chitosan der Formel (I), welches eine Viskosität von 15 bis 15.000 mPa*s aufweist. Bevorzugt wird die zuvor angeführte Viskosität mittels eines Brookfield RVDV II+, Spindel Nr. 2, 20 rpm, 20 °C unter Verwendung von 1 Gew.-% Chitosan der Formel (I) in einer 1 Gew.-%igen Essigsäurelösung, jeweils bezogen auf das Gesamtgewicht der Lösung, bestimmt. Als Lösung ist hierbei eine 1 Gew.-%ige Essigsäurelösung zu verstehen, in welcher 1 Gew.-% des Chitosans sowie 1 Gew.-% Essigsäure, bezogen auf das Gesamtgewicht dieser Lösung, gelöst sind.

Gemäß einer bevorzugten Ausführungsform der vorliegenden Erfindung steht in der Formel (I) y für ganze Zahlen von 1 bis 22.000, vorzugsweise von 1 bis 20.000, bevorzugt von 1 bis 19.000, weiter bevorzugt von 1 bis 18.000, insbesondere von 1 bis 17.500.

Weiterhin ist es im Rahmen der vorliegenden Erfindung bevorzugt, wenn in der Formel (I) x und z, jeweils unabhängig voneinander, für ganze Zahlen von 5 bis 20.000, vorzugsweise von 6 bis 15.200, bevorzugt von 7 bis 13.000, weiter bevorzugt von 8 bis 12.500, insbesondere von 10 bis 11.700, stehen. Die Verwendung von Chitosanen der Formel (I) mit den zuvor angeführten Zahlenwerten für x, y und z führt zu einer besonders effektiven Beeinflussung der Schweißdrüse(n) und somit zu einer besonders hohen Verminderung bzw. Reduzierung von Schweiß.

Besonders gute Ergebnisse werden im Rahmen der vorliegenden Erfindung erhalten, wenn Chitosane der Formel (I) eingesetzt werden, welche einen bestimmten Deacetylierungsgrad aufweisen. Bevorzugte erfindungsgemäße schweißhemmende kosmetische Mittel sind daher dadurch gekennzeichnet, dass das mindestens eine Chitosan der Formel (I) einen Deacetylierungsgrad von 70 bis 99 %, vorzugsweise von 80 bis 98 %, bevorzugt von 80 bis 95 %, weiter bevorzugt von 80 bis 92 %, insbesondere von 80 bis 90 %, aufweist. Der Deacetylierungsgrad kann beispielsweise mittels NMR-Spektroskopie bestimmt werden (Hwang K. T. et. al.; J. Agric. Food Chem.; 2002, 50, Seiten 1876 bis 1882). Chitosane, welche einen Deacetylierungsgrad von weniger als 70 % aufweisen, besitzen nur eine unzureichende Löslichkeit und eignen sich daher nicht für den Einsatz in den erfindungsgemäßen schweißhemmenden Mitteln, da aufgrund der Unlöslichkeit kein Eindringen des Chitosans in die Ausführungsgänge der Schweißdrüse(n) und somit keine Verminderung bzw. Reduzierung von Schweiß möglich ist.

Das in den erfindungsgemäßen schweißhemmenden kosmetischen Mitteln verwendete Chitosan der Formel (I) kann aus verschiedenen Quellen gewonnen werden. Bevorzugt ist es jedoch, wenn das mindestens eine Chitosan der Formel (I) aus maritimen Quellen, insbesondere Krabben, Garnelen, Krill, Pilzen, Zooplankton, Insekten, Mikroorganismen, modifizierte Mikroorganismen oder pflanzlichen Quellen isoliert ist. Bevorzugt wird das in den erfindungsgemäßen schweißhemmenden kosmetischen Mitteln eingesetzte Chitosan der Formel (I) aus maritimen Quellen, insbesondere aus dem Chitin von Krustentieren, welche in großen Mengen bei der Zubereitung von Krabben, Hummern, Garnelen etc. zu Speisezwecken anfallen, gewonnen. Das dort enthaltende Chitin wird üblicherweise durch den Zusatz von Basen deprotoniert, durch Zugabe von Mineralsäuren demineralisiert und anschließend unter Verwendung von starken Basen, wie beispielsweise konzentrierter Natronlauge, deacetyliert. Neben diesem Herstellverfahren kann das Chitosan der Formel (I) jedoch auch aus niederen Pilzen wie beispielsweise *Absidia sp., Rhizopus sp.* und *Mucor sp.,* welche Chitosan als Zellwandkomponente aufweisen, gewonnen werden. Darüber hinaus ist die Gewinnung von Chitosan der Formel (I) auch durch den Einsatz von gentechnisch modifizierten Mikroorganismen möglich, welche Chitosan mittels enzymatischer Prozesse synthetisieren. Weiterhin kann Chitosan aus chitosanhaltigen pflanzlichen Quellen gewonnen werden.

Im Rahmen der vorliegenden Erfindung ist es vorteilhaft, wenn die Chitosane der Formel (I) ein bestimmtes Molekulargewicht aufweisen. Bevorzugte erfindungsgemäße schweißhemmende kosmetische Mittel sind daher dadurch gekennzeichnet, dass das mindestens eine Chitosan der Formel (I) ein mittleres Molekulargewicht M_{w} von 5.000 bis 6.000.000 Da, vorzugsweise von 6.000 bis 5.500.000 Da, bevorzugt von 8.000 bis 5.050.000 Da, insbesondere von 10.000 bis 5.000.000 Da, aufweist. Das mittlere Molekulargewicht M_{w} kann beispielsweise durch Gelpermeationschromatographie (GPC) bestimmt werden (Hwang K. T. et. al.; J. Agric. Food Chem.; 2002, 50, Seiten 1876 bis 1882). Der Einsatz von Chitosanen mit den zuvor angeführten Molekulargewichten führt zu einer besonders effektiven Beeinflussung der Schweißdrüse(n) durch Ausfällung des Chitosans innerhalb der Schweißdrüse(n) bzw. durch Störung des Ladungsgleichgewichts innerhalb der Schweißdrüse(n) und somit zu einer hervorragenden schweißhemmenden Wirkung der erfindungsgemäßen kosmetischen Mittel.

Besonders bevorzugt werden erfindungsgemäß Chitosane der Formel (I) eingesetzt, welche eine bestimmte Viskosität aufweisen. Es ist daher im Rahmen der vorliegenden Erfindung bevorzugt, wenn das mindestens eine Chitosan der Formel (I) eine Viskosität von 15 bis 10.000 mPa*s, vorzugsweise von 15 bis 8.000 mPa*s, bevorzugt von 15 bis 6.000 mPa*s, insbesondere von 15 bis 5.000 mPa*s, aufweist, wobei die Viskosität jeweils mit Brookfield RVDV II+, Spindel Nr. 2, 20 rpm, 20 °C bestimmt wird und wobei zur Bestimmung der Viskosität 1 Gew.-% Chitosan der Formel (I) in einer 1 Gew.-%igen Essigsäurelösung, jeweils bezogen auf das Gesamtgewicht der Lösung, verwendet wird. Chitosane, welche eine Viskosität unterhalb von 15 mPa*s aufweisen, resultieren nicht in der gewünschten Beeinflussung der Schweißdrüse(n). Dahingegen führen Chitosane mit einer Viskosität von mehr als 15.000 mPa*s aufgrund der hohen Viskosität zu Formulierungs- und Anwendungsproblemen der erfindungsgemäßen kosmetischen Mittel und sind daher im Rahmen der vorliegenden Erfindung ebenfalls nicht geeignet.

Eine besonders effektive Verminderung des Achselschweißes durch das mindestens eine Chitosan der Formel (I) wird im Rahmen der vorliegenden Erfindung erreicht, wenn das mindestens eine Chitosan der Formel (I) in einer Gesamtmenge von 0,05 bis 40 Gew.-%, vorzugsweise von 0,1 bis 35 Gew.-%, bevorzugt von 0,2 bis 30 Gew-%, weiter bevorzugt von 0,4 bis 25 Gew.-%, insbesondere von 0,5 bis 20 Gew.-%, bezogen auf das Gesamtgewicht des treibmittelfreien schweißhemmenden kosmetischen Mittels, enthalten ist. Ohne sich auf diese Theorie beschränken zu wollen resultiert der Einsatz der vorstehend genannten Mengen des mindestens einen speziellen Chitosans in einer signifikanten Beeinflussung der Schweißdrüse(n) durch Ausfällung des Chitosans in den Ausführungsgängen der Schweißdrüsen oder durch Beeinflussung des Ladungsgleichgewichts innerhalb der Schweißdrüse(n). Auf diese Weise wird eine hervorragende schweißhemmende Wirkung gewährleistet. Weiterhin führt der Einsatz der zuvor genannten Mengen des mindestens einen speziellen Chitosans nicht zu instabilen Formulierungen, so dass die Stabilität der erfindungsgemäßen schweißhemmenden kosmetischen Mittel selbst über lange Lagerungszeiträume gewährleistet wird.

Gemäß einer weiteren bevorzugten Ausführungsform der vorliegenden Erfindung weist das schweißhemmende kosmetische Mittel einen pH-Wert von pH 2 bis pH 6 auf. Innerhalb dieses Bereichs ist eine stabile Formulierung der erfindungsgemäßen kosmetischen Mittel möglich, ohne dass unerwünschte Wechselwirkungen zwischen den Inhaltsstoffen auftreten. Die Einstellung des gewünschten pH-Wertes kann erfindungsgemäß durch Verwendung von dem Fachmann bekannten und in schweißhemmenden kosmetischen Mitteln üblichen Säuren und Basen erfolgen.

Erfindungsgemäß ist es weiterhin bevorzugt, wenn das schweißhemmende kosmetische Mittel zusätzlich mindestens ein Konservierungsmittel enthält. Erfindungsgemäß bevorzugte Konservierungsmittel sind Formaldehydabspalter lodopropinylbutylcarbamate, Parabene, Phenoxyethanol, Ethanol, Benzoesäure und deren Salze, Dibromdicyanobutan, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol, Salicylsäure und Salicylate. Weitere im Rahmen der vorliegenden Erfindung einsetzbare Konservierungsmittel sind die in Anlage 6 der Kosmetikverordnung aufgeführten Substanzen sowie kosmetische Rohstoffe mit konservierenden Eigenschaften oder Rohstoffe, welche die konservierende Wirkung der vorgenannten Konservierungsmittel unterstützen bzw. verstärken. Die Konservierungsmittel sind vorzugsweise in einer Gesamtmenge von 0,01 bis 10 Gew.-%, vorzugsweise von 0,1 bis 7 Gew.-%, bevorzugt von 0,2 bis 5 Gew.-%, insbesondere von 0,3 bis 2,0 Gew.-%, bezogen auf das Gesamtgewicht des treibmittelfreien schweißhemmenden kosmetischen Mittels, enthalten.

Im Rahmen der vorliegenden Erfindung ist es bevorzugt, wenn das schweißhemmende kosmetische Mittel als Wasser-in-ÖI-Emulsion vorliegt. Hierbei kann es sich insbesondere um eine versprühbare Wasser-in-ÖI-Emulsion, welche mittels eines Treibmittels versprüht werden kann, oder um eine gelartige Wasser-in-ÖI-Emulsion, welche mittels einem Spender appliziert werden kann, handeln. In diesem Zusammenhang ist es bevorzugt, wenn das in Form einer Wasser-in-ÖI-Emulsion vorliegende erfindungsgemäße schweißhemmende kosmetische Mittel das mindestens eine Chitosan der Formel (I) in einer Gesamtmenge von 0,05 bis 40 Gew.-%, vorzugsweise von 0,1 bis 35 Gew.-%, bevorzugt von 0,2 bis 30 Gew-%, weiter bevorzugt von 0,4 bis 25 Gew.-%, insbesondere von 0,5 bis 20 Gew.-%, bezogen auf das Gesamtgewicht des treibmittelfreien schweißhemmenden kosmetischen Mittels, enthält.

Es kann jedoch erfindungsgemäß gleichermaßen bevorzugt sein, wenn das schweißhemmende kosmetische Mittel als ÖI-in-Wasser-Emulsion vorliegt. In diesem Fall wird das erfindungsgemäße kosmetische Mittel bevorzugt als treibmittelhaltiges Aerosol, treibmittelfreies Pumpspray oder Quetschspray versprüht oder als Roll-on appliziert. In diesem Zusammenhang ist es bevorzugt, wenn das in Form einer ÖI-in-Wasser-Emulsion vorliegende schweißhemmende kosmetische Mittel das mindestens eine Chitosan der Formel (I) in einer Gesamtmenge von 0,05 bis 40 Gew.-%, vorzugsweise von 0,1 bis 35 Gew.-%, bevorzugt von 0,2 bis 30 Gew-%, weiter bevorzugt von 0,4 bis 25 Gew.-%, insbesondere von 0,5 bis 20 Gew.-%, enthält.

Gemäß einer weiteren bevorzugten Ausführungsform der vorliegenden Erfindung können die erfindungsgemäßen kosmetischen Mittel nur einen geringen Gehalt an freiem Wasser bzw. kein freies Wasser enthalten. Unter freiem Wasser im Sinne der vorliegenden Erfindung wird Wasser verstanden, welches von Kristallwasser, Hydratationswasser oder ähnlich molekular gebundenem Wasser der eingesetzten Bestandteile verschieden ist. Das schweißhemmende kosmetische Mittel enthält bevorzugt freies Wasser in einer Gesamtmenge von weniger als 10 Gew.-%, vorzugweise von weniger als 8 Gew.-%, bevorzugt von weniger als 5 Gew.-%, weiter bevorzugt von weniger als 3 Gew.-%, noch mehr bevorzugt von weniger als 1 Gew.-%, insbesondere von 0 Gew.-%, bezogen auf das Gesamtgewicht des treibmittelfreien schweißhemmenden kosmetischen Mittels.

Im Rahmen einer weiteren Ausführungsform ist es jedoch erfindungsgemäß ebenfalls bevorzugt, wenn das schweißhemmende kosmetische Mittel als wässrige, wässrig-alkoholische oder wässrig-glykolische Lösung vorliegt. Da die erfindungsgemäßen kosmetischen Mittel keine schweißhemmenden Halogenide und/oder Hydroxyhalogenide von Aluminium und/oder Zirconium enthalten, welche durch den Zusatz protischer Lösungsmittel eine verminderte schweißhemmende Wirkung aufweisen, können erfindungsgemäß protische Lösungsmittel, wie wässrige Lösungen, zur Formulierung der erfindungsgemäßen schweißhemmenden kosmetischen Mittel verwendet werden, ohne dass eine signifikante Verringerung der Antitranspirantwirkung auftritt. Daher gewährleistet der Zusatz des mindestens einen speziellen Chitosans selbst bei Verwendung von protischen Lösungsmitteln eine effektive Beeinflussung der der Schweißdrüse(n) und somit eine hervorragende Antitranspirantwirkung.

Im Zusammenhang mit dieser Ausführungsform der vorliegenden Erfindung wurde überraschenderweise festgestellt, dass die Beeinflussung der Schweißdrüse(n) durch das mindestens eine spezielle Chitosan der Formel (I) signifikant gesteigert werden kann, wenn die erfindungsgemäßen schweißhemmenden kosmetischen Mittel freies Wasser in einer Menge von 5 bis 99 Gew.-%, bezogen auf das Gesamtgewicht des treibmittelfreien schweißhemmenden kosmetischen Mittels, enthalten. In einer besonders bevorzugten Ausführungsform der vorliegenden Erfindung enthält das schweißhemmende kosmetische Mittel daher freies Wasser in einer Gesamtmenge von 5 bis 96 Gew.-%, vorzugsweise von 15 bis 80 Gew.-%, bevorzugt von 30 bis 70 Gew.-%, insbesondere von 40 bis 60 Gew.-%, bezogen auf das Gesamtgewicht des treibmittelfreien schweißhemmenden kosmetischen Mittels.

Weiterhin ist es im Zusammenhang mit dieser Ausführungsform bevorzugt, wenn das schweißhemmende kosmetische Mittel Ethanol in einer Gesamtmenge von 1 bis 99 Gew.-%, vorzugsweise von 5 bis 70 Gew.-%, bevorzugt von 7 bis 50 Gew.-%, insbesondere von 10 bis 30 Gew.-%, bezogen auf das Gesamtgewicht des treibmittelfreien schweißhemmenden kosmetischen Mittels, enthält. Wie zuvor ausgeführt, können durch den Einsatz des mindestens einen speziellen Chitosans der Formel (I) selbst hohe Mengen an protischen Lösungsmitteln, wie Ethanol, verwendet werden, ohne dass die Antitranspirantwirkung des erfindungsgemäßen schweißhemmenden kosmetischen Mittels negativ beeinflusst wird.

Die Applikation des erfindungsgemäßen schweißhemmenden kosmetischen Mittels kann mittels verschiedener Verfahren erfolgen. Gemäß einer bevorzugten Ausführungsform ist das schweißhemmende kosmetische Mittel als Spray-Applikation konfektioniert. Die Spray-Applikation erfolgt mit einer Sprühvorrichtung, welche in einem Behälter eine Füllung aus dem erfindungsgemäßen flüssigen, viskos-fließfähigen, suspensionsförmigen oder pulverförmigen schweißhemmenden kosmetischen Mittel enthält. Die Füllung kann unter dem Druck eines Treibmittels stehen (Druckgasdosen, Druckgaspackungen, Aerosolpackungen), oder es kann sich um einen mechanisch zu bedienenden Pumpzerstäuber ohne Treibgas (Pumpsprays/ Quetschflasche) handeln. Die Zerstäubung des schweißhemmenden kosmetischen Mittels kann hierbei physikalisch, mechanisch oder elektromechanisch, beispielsweise durch Piezzoeffekte oder elektrische Pumpen, erfolgen. Im Rahmen dieser Ausführungsform einsetzbare Behälter und Entnahmevorrichtungen sind beispielsweise in der Offenlegungsschrift DE 10 2012 222 692 A1 beschrieben.

Das schweißhemmende kosmetische Mittel kann weiterhin bevorzugt als Stift, Soft Solid, Creme, Gel, Roll-on, loses oder kompaktes Puder konfektioniert sein. Die Formulierung der erfindungsgemäßen schweißhemmenden kosmetischen Mittel in einer bestimmten Darreichungsform, wie beispielsweise einem Antitranspirant-Roll-on, einem Antitranspirantstift oder einem Antitranspirantgel, richtet sich bevorzugt nach den Anforderungen des Verwendungszwecks. Je nach Verwendungszweck können die erfindungsgemäßen schweißhemmenden kosmetischen Mittel daher in fester, halbfester, flüssiger, disperser, emulgierter, suspendierter, gelförmiger, mehrphasiger oder puderförmiger Form vorliegen. Unter den Begriff der Flüssigkeit fallen im Sinne der vorliegenden Erfindung auch jegliche Arten von Festkörperdispersionen in Flüssigkeiten. Weiterhin werden unter mehrphasigen erfindungsgemäßen schweißhemmenden kosmetischen Mitteln im Sinne der vorliegenden Erfindung Mittel verstanden, welche mindestens 2 verschiedene Phasen mit einer Phasentrennung aufweisen und bei welchen die Phasen horizontal, also übereinander, oder vertikal, also nebeneinander, angeordnet sein können. Die Applikation kann beispielsweise mit einem Rollkugelapplikator oder mittels eines festen Stifts erfolgen.

Es kann im Rahmen der vorliegenden Erfindung ebenfalls bevorzugt sein, wenn das schweißhemmende kosmetische Mittel auf und/oder in einem wegwerfbaren Substrat, ausgewählt aus der Gruppe von Tüchern, Pads und Bauschen, enthalten ist. Besonders bevorzugt sind Feuchttücher, d.h. für den Anwender vorgefertigte, bevorzugt einzeln abgepackte, Feuchttücher, wie sie z. B. aus dem Bereich der Glasreinigung oder aus dem Bereich der feuchten Toilettenpapiere wohlbekannt sind. Solche Feuchttücher, die vorteilhafter Weise auch Konservierungsstoffe enthalten können, sind mit einem erfindungsgemäßen schweißhemmenden kosmetischen Mittel imprägniert oder beaufschlagt und bevorzugt einzeln verpackt. Bevorzugte Substratmaterialien sind ausgewählt aus porösen flächigen Tüchern. Zu diesen Tüchern gehören Tücher aus gewebten und ungewebten (Vlies) synthetischen und natürlichen Fasern, Filz, Papier oder Schaumstoff, wie hydrophilem Polyurethanschaum. Erfindungsgemäß bevorzugte deodorierende oder schweißhemmende Substrate können durch Tränken oder Imprägnierung oder auch durch Aufschmelzen eines erfindungsgemäßen schweißhemmenden kosmetischen Mittels auf ein Substrat erhalten werden.

Das erfindungsgemäße schweißhemmende kosmetische Mittel kann neben den zuvor angeführten Verbindungen weitere Wirk- und Inhaltsstoffe enthalten.

Erfindungsgemäß bevorzugt enthält das schweißhemmende kosmetische Mittel daher mindestens einen weiten Hilfsstoff, ausgewählt aus der Gruppe von (i) Emulgatoren und/oder Tensiden; (ii) Verdickungsmitteln; (iii) Chelatbildnern; (iv) Deodorant-Wirkstoffen; (v) ein- und/oder mehrwertigen Alkoholen und/oder Polyethylenglycolen; (vi) hautkühlenden Wirkstoffen; (vii) pH-Stellmitteln; (viii) hautpflegenden Wirkstoffen, wie Moisturizern, hautberuhigenden Stoffen, hautaufhellenden Stoffen, hautglättenden Stoffen; sowie (ix) deren Mischungen.

Erfindungsgemäß bevorzugt geeignete Emulgatoren und Tenside sind ausgewählt aus anionischen, kationischen, nichtionischen, amphoteren, insbesondere ampholytischen und zwitterionischen Emulgatoren und Tensiden. Tenside sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8 bis 28 Kohlenstoffatomen, die gesättigt oder ungesättigt, linear oder verzweigt sein kann. Besonders bevorzugt ist diese C₈-C₂₈-Alkylkette linear. Im Rahmen der vorliegenden Erfindung bevorzugt einsetzbare Emulgatoren und Tenside sind beispielsweise in den Offenlegungsschriften DE 10 2012 222 692 A1, DE 10 2010 063 250 A1 sowie DE 10 2010 055 816 A1 offenbart.

Zur Verdickung der erfindungsgemäßen schweißhemmenden kosmetischen Mittel werden bevorzugt Substanzen eingesetzt, welche ausgewählt sind aus Celluloseethern, Xanthan-Gum, Sclerotium Gum, Succinoglucanen, Polygalactomannanen, Pectinen, Agar, Carragheen (Carrageenan), Traganth, Gummi arabicum, Karayagummi, Taragummi, Gellan, Gelatine, Propylenglycolalginat, Alginsäuren und deren Salze, Polyvinylpyrrolidonen, Polyvinylalkoholen, Polyacrylamiden, physikalisch (z. B. durch Vorverkleisterung) und/oder chemisch modifizierten Stärken, Acrylsäure-Acrylat-Copolymeren, Acrylsäure-Acrylamid-Copolymeren, Acrylsäure-Vinylpyrrolidon-Copolymeren, Acrylsäure-Vinylformamid-Copolymeren und Polyacrylaten. Besonders bevorzugte Verdickungsmittel sind weiterhin ausgewählt aus Carbomeren. Carbomere sind verdickende vernetzte Polymere aus Acrylsäure, Methacrylsäure sowie deren Salzen. Die Vernetzung kann mittels polyfunktioneller Verbindungen wie Polyalkylenether von Polysacchariden oder Polyalkoholen, beispielsweise Sucroseallylether, Pentaerythritolallylether, Propylenallylether, erfolgen. Bevorzugt im Rahmen der vorliegenden Erfindung sind Homopolymere von Acrylsäure oder deren Salzen, welche mit einem Pentaerythritolallylether, einem Sucroseallylether oder einem Propylenallylether vernetzt sind. Ein im Rahmen der vorliegenden Erfindung einsetzbares Verdickungsmittel ist ein Copolymer aus C₁₀₋₃₀-Alkylacrylat, Acrylsäure, Methacrylsäure sowie deren Estern, welches mit einem Sucroseallylether oder einem Pentaerythritolallylether vernetzt ist. Verdickungsmittel auf Basis von Carbomeren sind die unter dem Handelsnamen Carbopol® (BF Goodrich, Ohio, USA) erhältlichen Produkte wie beispielsweise Carbopol 934, Carbopol 940, Carbopol 941, Carbopol 971, Carbopol 974, Carbopol EZ2, Carbopol ETD 2001, Carbopol ETD 2020, Carbopol ETD 2050, Carbopol ultrez 10, Carbopol ultrez 20, oder Carbopol ultrez 21.

Weiterhin können zur Verdickung der erfindungsgemäßen schweißhemmenden kosmetischen Mittel lipophile Verdickungsmittel eingesetzt werden. Erfindungsgemäß bevorzugte lipophile Verdickungsmittel sind ausgewählt aus hydrophobierten Tonmineralien, Bentoniten, pyrogenen Kieselsäuren sowie deren Derivaten.

Um die Beeinflussung der Schweißdrüse(n) durch das mindestens eine spezielle Chitosan der Formel (I) weiter zu unterstützen, kann es von Vorteil sein, den erfindungsgemäßen schweißhemmenden kosmetischen Mitteln mindestens einen Chelatbildner, in einer Gesamtmenge von 0,01 bis 3,0 Gew.-%, vorzugsweise von 0,02 bis 1,0 Gew.-%, insbesondere von 0,05 bis 0,1 Gew.-%, bezogen auf das Gesamtgewicht des treibmittelfreien erfindungsgemäßen schweißhemmenden Mittels, zuzusetzen. Im Rahmen der vorliegenden Erfindung sind bevorzugte Chelatbildner ausgewählt aus der Gruppe von β-Alanindiessigsäure, Cyclodextrin, Diethylentriaminpentamethylenphosphonsäure, Natrium-, Kalium-, Calciumdinatrium-, Ammonium- und Triethanolaminsalzen der Ethylendiamintetraessigsäure (EDTA), Etidronsäure, Hydroxyethylethylendiamintetraessigsäure (HEDTA) und ihren Natriumsalzen, Natriumsalzen der Nitrilotriessigsäure (NTA), Diethylentriaminpentaessigsäure, Phytinsäure, Hydroxypropylcyclodextrin, Methylcyclodextrin, Aminotrimethylenphosphonat-Pentanatrium, Ethylendiamintetramethylenphosphonat-Pentanatrium, Diethylentriamin-pentaacetat-Pentanatrium, Pentanatriumtriphosphat, Kalium-EDTMP, Natrium-EDTMP, Natriumdihydroxyethylglycinat, Natriumphytat, Natriumpolydimethylglycinophenolsulfonat, Tetrahydroxyethylethylendiamin, Tetrahydroxypropylethylendiamin, Tetrakaliumetidronat, Tetranatriumetidronat, Tetranatriumiminodisuccinat, Trinatriumethylendiamindisuccinat, Tetranatrium-N,N-bis(Carboxymethyl)glutamat, Tetranatrium-DL-Alanin-N,N-diacetat und Desferrioxamin.

Die desodorierende Wirkung der erfindungsgemäßen schweißhemmenden kosmetischen Mittel kann weitergehend gesteigert werden, wenn zusätzlich mindestens ein Deodorant-Wirkstoff mit antibakterieller und/oder bakteriostatischer und/oder enzyminhibierender und/oder geruchsneutralisierender und/oder geruchsabsorbierender Wirkung in einer Gesamtmenge von 0,0001 bis 40 Gew.-%, vorzugsweise von 0,2 bis 20 Gew.-%, bevorzugt von 1 bis 15 Gew.-%, insbesondere von 1,5 bis 5 Gew.-%, bezogen auf das Gesamtgewicht des treibmittelfreien erfindungsgemäßen schweißhemmenden kosmetischen Mittels, enthalten ist. Sofern Ethanol in den erfindungsgemäßen Mitteln eingesetzt wird, gilt dieses im Rahmen der vorliegenden Erfindung nicht als Deodorant-Wirkstoff, sondern als Bestandteil des Trägers. Erfindungsgemäß bevorzugte Deodorant-Wirkstoffe sind beispielsweise in der Offenlegungsschrift DE 10 2010 063 250 A1 offenbart.

Bevorzugte erfindungsgemäße schweißhemmenden kosmetische Mittel enthalten weiterhin mindestens ein wasserlösliches mehrwertiges C₂₋₉-Alkanol mit 2 bis 6 Hydroxylgruppen und/oder mindestens ein wasserlösliches Polyethylenglycol mit 3 bis 50 Ethylenoxid-Einheiten sowie Mischungen hiervon. Hierunter fallen nicht die vorstehend erwähnten Deodorant-Wirkstoffe in Form von 1,2-Alkandiolen. Bevorzugte Alkanole und wasserlösliche Polyethylenglycole sind beispielsweise in der Offenlegungsschrift DE 10 2010 063 250 A1 beschrieben.

Gemäß einer weiteren Ausführungsform der vorliegenden Erfindung enthalten die schweißhemmenden kosmetischen Mittel weiterhin mindestens einen hautkühlenden Wirkstoff. Erfindungsgemäß geeignete hautkühlende Wirkstoffe sind beispielsweise Menthol, Isopulegol sowie Mentholderivate, z. B. Menthyllactat, Menthylglycolat, Menthyl Ethyl Oxamate, Menthylpyrrolidoncarbonsäure, Menthylmethylether, Menthoxypropandiol, Menthonglycerinacetal (9-Methyl-6-(1-methylethyl)-1,4-dioxaspiro (4.5)decan-2-methanol), Monomenthylsuccinat, 2-Hydroxymethyl-3,5,5-trimethylcyclohexanol und 5-Methyl-2-(1-methylethyl)cyclohexyl-N-ethyloxamat. Als hautkühlende Wirkstoffe bevorzugt sind Menthol, Isopulegol, Menthyllactat, Menthoxypropandiol, Menthylpyrrolidoncarbonsäure und 5-Methyl-2-(1-methylethyl)cyclohexyl-N-ethyloxamat sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol.

Als pH-Stellmittel kommen erfindungsgemäß bevorzugt Säuren und/oder Alkalisierungsmittel und/oder Puffer zum Einsatz. Als Säuren werden erfindungsgemäß bevorzugt anorganische Säuren (wie beispielsweise Salzsäure, Schwefelsäure oder Phosphorsäure) oder organische Säuren (wie beispielsweise Zitronensäure, Gluconsäure, Weinsäure oder Apfelsäure) eingesetzt. Die erfindungsgemäß verwendbaren Alkalisierungsmittel werden bevorzugt ausgewählt aus der Gruppe, welche gebildet wird, aus Ammoniak, basischen Aminosäuren, Alkalihydroxiden, Carbonaten und Hydrogencarbonaten, Alkanolaminen, beispielsweise Amino-2-methyl-1-propanol, Monoethanolamin, Triethanolamin, Diethanolamin und Triisopropanolamin, Alkalimetallmetasilikaten, Harnstoff, Morpholin, N-Methylglucamin, Imidazol, Alkaliphosphaten und Alkalihydrogenphosphaten. Als Alkalimetallionen dienen bevorzugt Lithium, Natrium, Kalium, insbesondere Natrium oder Kalium. Als Puffersysteme eignen sich im Rahmen der vorliegenden Erfindung insbesondere Kohlensäure-Bicarbonat-Puffer, Kohlensäure-Silicat-Puffer, Essigsäure-Acetat-Puffer, Phosphatpuffer, Ammoniakpuffer, Citronensäure- oder Citratpuffer, Puffer auf Basis von Tris(hydroxymethyl)-aminomethan, Puffer auf Basis von 4-(2-Hydroxyethyl)-1-piperazinethanesulfonsäure, Puffer auf Basis von 4-(2-Hydroxyethyl)-piperazin-1-propansulfonsäure, Puffer auf Basis von 2-(N-Morpholino)ethansulfonsäure sowie Barbital-Acetat-Puffer. Die Wahl des entsprechenden Puffersystems richtet sich hierbei nach dem gewünschten pH-Wert der erfindungsgemäßen schweißhemmenden kosmetischen Mittel.

In einer bevorzugten Ausführungsform sind die erfindungsgemäßen schweißhemmenden kosmetischen Mittel dadurch gekennzeichnet, dass sie - bezogen auf das Gesamtgewicht des treibmittelfreien schweißhemmenden kosmetischen Mittels -
- mindestens ein Chitosan der Formel (I) in einer Gesamtmenge von 0,05 bis 40 Gew.-%, vorzugsweise von 0,1 bis 35 Gew.-%, bevorzugt von 0,2 bis 30 Gew-%, weiter bevorzugt von 0,4 bis 25 Gew.-%, insbesondere von 0,5 bis 20 Gew.-%,
- 12 bis 98 Gew.-%, vorzugsweise 25 bis 55 Gew.-%, bevorzugt 30 bis 50 Gew.-%, insbesondere 35 bis 45 Gew.-% Wasser,
- mindestens einen Emulgator und/oder ein Tensid,
- mindestens ein pH-Stellmittel,
- mindestens ein Konservierungsmittel und
- mindestens einen Stoff, ausgewählt aus der Gruppe von kosmetischen Ölen, welche bei 20 °C und 1.013 hPa flüssig sind, Riechstoffen und Wachsen enthalten.

In einer weiteren bevorzugten Ausführungsform sind die erfindungsgemäßen schweißhemmenden kosmetischen Mittel dadurch gekennzeichnet, dass sie - bezogen auf das Gesamtgewicht des treibmittelfreien erfindungsgemäßen schweißhemmenden kosmetischen Mittels -
- mindestens ein Chitosan der Formel (I) in einer Gesamtmenge von 0,05 bis 40 Gew.-%, vorzugsweise von 0,1 bis 35 Gew.-%, bevorzugt von 0,2 bis 30 Gew-%, weiter bevorzugt von 0,4 bis 25 Gew.-%, insbesondere von 0,5 bis 20 Gew.-%,
- 12 bis 98 Gew.-%, vorzugsweise 25 bis 55 Gew.-%, bevorzugt 30 bis 50 Gew.-%, insbesondere 35 bis 45 Gew.-% Wasser,
- mindestens einen Emulgator und/oder ein Tensid,
- mindestens ein pH-Stellmittel,
- mindestens ein Konservierungsmittel,
- 0,01 bis 5 Gew.-%, vorzugsweise 0,1 bis 2 Gew.-%, bevorzugt 0,2 bis 0,7 Gew.-%, insbesondere 0,3 bis 0,5 Gew.-% eines Verdickungsmittels, und
- mindestens einen Stoff, ausgewählt aus der Gruppe von kosmetischen Ölen, welche bei 20 °C und 1.013 hPa flüssig sind, Riechstoffen und Wachsen enthalten.

In einer bevorzugten Ausführungsform sind die erfindungsgemäßen schweißhemmenden kosmetischen Mittel dadurch gekennzeichnet, dass sie - bezogen auf das Gesamtgewicht des treibmittelfreien erfindungsgemäßen schweißhemmenden kosmetischen Mittels -
- mindestens ein Chitosan der Formel (I) in einer Gesamtmenge von 0,05 bis 40 Gew.-%, vorzugsweise von 0,1 bis 35 Gew.-%, bevorzugt von 0,2 bis 30 Gew-%, weiter bevorzugt von 0,4 bis 25 Gew.-%, insbesondere von 0,5 bis 20 Gew.-%,
- 12 bis 98 Gew.-%, vorzugsweise 25 bis 55 Gew.-%, bevorzugt 30 bis 50 Gew.-%, insbesondere 35 bis 45 Gew.-% Wasser,
- mindestens ein Treibmittel in einer Gesamtmenge von 1 bis 98 Gew.-%, vorzugsweise von 20 bis 90 Gew.-%, bevorzugt von 30 bis 85 Gew-%, insbesondere von 40 bis 75 Gew.-%,
- mindestens einen Emulgator und/oder ein Tensid,
- mindestens ein pH-Stellmittel,
- mindestens ein Konservierungsmittel und
- mindestens einen Stoff, ausgewählt aus der Gruppe von kosmetischen Ölen, welche bei 20 °C und 1.013 hPa flüssig sind, Riechstoffen und Wachsen enthalten.

In einer weiteren bevorzugten Ausführungsform sind die erfindungsgemäßen schweißhemmenden kosmetischen Mittel dadurch gekennzeichnet, dass sie - bezogen auf das Gesamtgewicht des treibmittelfreien erfindungsgemäßen schweißhemmenden kosmetischen Mittels -
- mindestens ein Chitosan der Formel (I) in einer Gesamtmenge von 0,05 bis 40 Gew.-%, vorzugsweise von 0,1 bis 35 Gew.-%, bevorzugt von 0,2 bis 30 Gew-%, weiter bevorzugt von 0,4 bis 25 Gew.-%, insbesondere von 0,5 bis 20 Gew.-%
- 12 bis 98 Gew.-%, vorzugsweise 25 bis 55 Gew.-%, bevorzugt 30 bis 50 Gew.-%, insbesondere 35 bis 45 Gew.-% Wasser,
- mindestens ein Treibmittel in einer Gesamtmenge von 1 bis 98 Gew.-%, vorzugsweise von 20 bis 90 Gew.-%, bevorzugt von 30 bis 85 Gew-%, insbesondere von 40 bis 75 Gew.-%,
- mindestens einen Emulgator und/oder ein Tensid,
- mindestens ein pH-Stellmittel,
- mindestens ein Konservierungsmittel,
- 0,01 bis 5 Gew.-%, vorzugsweise 0,1 bis 2 Gew.-%, bevorzugt 0,2 bis 0,7 Gew.-%, insbesondere 0,3 bis 0,5 Gew.-% eines Verdickungsmittels, und
- mindestens einen Stoff, ausgewählt aus der Gruppe von kosmetischen Ölen, welche bei 20 °C und 1.013 hPa flüssig sind, Riechstoffen und Wachsen enthalten.

Im Rahmen der vorliegenden Erfindung kann es auch vorgesehen sein, das erfindungsgemäße kosmetische Mittel als Zwei-Komponenten-Mittel zu konfektionieren. Die einzelnen Komponenten werden hierzu vorzugsweise in getrennten Containern gelagert und in beliebiger Reihenfolge nacheinander oder gleichzeitig auf die Haut aufgetragen. Eine Auftrennung in Mehrkomponentensysteme ist insbesondere dort bevorzugt, wo Inkompatibilitäten der Inhaltsstoffe zu erwarten oder zu befürchten sind.

Ein weiterer Gegenstand der vorliegenden Erfindung ist daher eine Verpackungseinheit (Kit-of-Parts), umfassend - getrennt voneinander konfektioniert -
a) mindestens einen ersten Container (C1), enthaltend ein kosmetisches Mittel (M1) umfassend mindestens einen schweißhemmenden Wirkstoff, und
b) mindestens einen zweiten Container (C2), enthaltend ein kosmetisches Mittel (M2) umfassend mindestens ein Chitosan der Formel (I) 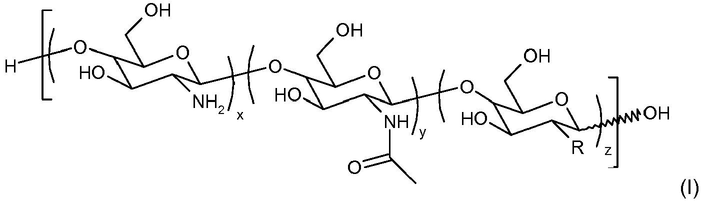 worin
   x und z, jeweils unabhängig voneinander, für ganze Zahlen von 5 bis 25.000 stehen,
   y für ganze Zahlen von 1 bis 25.000 steht,
   R für eine NH₂-Gruppe oder *-NH₃⁺CH₃CH(OH)C(O)O⁻ oder *-NH₃⁺OHCH₂C(O)O⁻ steht,
   mit der Maßgabe, dass die Reihenfolge der in der eckigen Klammer enthaltenden Einheiten mit den Indices x, y und z beliebig ist und dass der Rest R über * gebunden ist, und wobei das kosmetische Mittel (M2) keine aluminiumhaltigen Verbindungen enthält.

Unter dem Begriff "schweißhemmender Wirkstoff" werden erfindungsgemäß Wirkstoffe verstanden, welche die Transpiration der Schweißdrüsen des Körpers vermindern bzw. reduzieren. Hierunter fallen jedoch nicht die in dem kosmetischen Mittel (M2) enthaltenen Chitosane der Formel (I).

Bezüglich weiterer Ausführungsformen der erfindungsgemäßen Verpackungseinheit gilt *mutatis mutandis* das zu den erfindungsgemäßen kosmetischen Mitteln Gesagte.

Ein weiterer Gegenstand der vorliegenden Erfindung ist die Verwendung mindestens eines Chitosans zur zumindest teilweisen Beeinflussung der Schweißdrüse(n), wobei das Chitosan die Formel (I) aufweist, 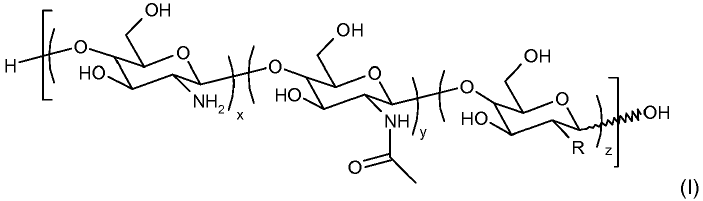 worin
x und z, jeweils unabhängig voneinander, für ganze Zahlen von 5 bis 25.000 stehen,
y für ganze Zahlen von 1 bis 25.000 steht,
R für eine NH₂-Gruppe oder *-NH₃⁺CH₃CH(OH)C(O)O⁻ oder *-NH₃⁺OHCH₂C(O)O⁻ steht, mit der Maßgabe, dass die Reihenfolge der in der eckigen Klammer enthaltenden Einheiten mit den Indices x, y und z beliebig ist und dass der Rest R über * gebunden ist.

Unter Beeinflussung der Schweißdrüse(n) ist erfindungsgemäß die Beeinflussung der Schweißdrüse(n) dahingehend zu verstehen, dass die Absonderung von Schweiß aus dem Ausführungsgang vermieden bzw. verringert wird. Ohne sich auf eine Theorie beschränken zu wollen kann dies einerseits durch Bildung eines Niederschlags des mindestens einen speziellen Chitosans der Formel (I) in dem Ausführungsgang der Schweißdrüse bzw. den Ausführungsgängen der Schweißdrüsen geschehen. Andererseits kann dies jedoch auch durch Störung des Ladungsgleichgewichts innerhalb der Ausführungsgänge der Schweißdrüsen geschehen.

Bezüglich weiterer Ausführungsformen der erfindungsgemäßen Verwendung gilt *mutatis mutandis* das zu den erfindungsgemäßen schweißhemmenden kosmetischen Mitteln Gesagte.

Darüber hinaus ist ein weiterer Gegenstand der vorliegenden Erfindung die Verwendung einer Kombination, enthaltend
a) mindestens einen Stoff, ausgewählt aus der Gruppe von kosmetischen Ölen, welche bei 20 °C und 1.013 hPa flüssig sind, Riechstoffen und Wachsen,
b) Treibmittel in einer Gesamtmenge von 0 bis 99 Gew.-%, bezogen auf das Gesamtgewicht des schweißhemmenden kosmetischen Mittels, und
c) mindestens ein Chitosan mit einer Viskosität von 15 bis 15.000 mPa*s, wobei das Chitosan die Formel (I) aufweist 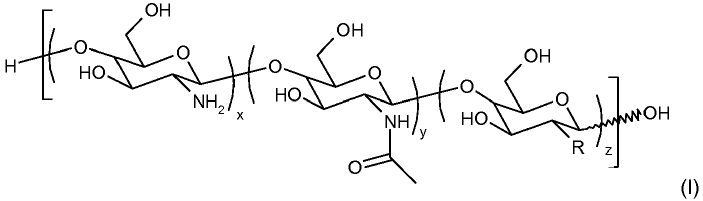 worin
   x und z, jeweils unabhängig voneinander, für ganze Zahlen von 5 bis 25.000 stehen,
   y für ganze Zahlen von 1 bis 25.000 steht,
   R für eine NH₂-Gruppe oder *-NH₃⁺CH₃CH(OH)C(O)O⁻ oder *-NH₃⁺OHCH₂C(O)O⁻ steht, mit der Maßgabe, dass die Reihenfolge der in der eckigen Klammer enthaltenden Einheiten mit den Indices x, y und z beliebig ist und dass der Rest R über * gebunden ist, und
d) keine aluminiumhaltigen Verbindungen,
   zur Reduzierung und/oder Verhinderung von Schweiß, insbesondere Achselschweiß oder Schweiß anderer Körperregionen.

Der Begriff der "Kombination" im Sinne der vorliegenden Erfindung umfasst eine Mischung der oben angegebenen Inhaltsstoffe a), b) und c). Bezüglich weiterer bevorzugter Ausführungsformen der Verwendung der vorstehend genannten Kombination gilt *mutatis mutandis* das zu den erfindungsgemäßen schweißhemmenden kosmetischen Mitteln sowie zu der erfindungsgemäßen Verwendung Gesagte.

Schließlich ist ein weiterer Gegenstand der vorliegenden Erfindung ein nicht-therapeutisches kosmetisches Verfahren zur Verhinderung und/oder Reduzierung der Transpiration des Körpers, bei welchem ein erfindungsgemäßes schweißhemmendes kosmetisches Mittel oder die kosmetischen Mittel (M1) und (M2) der erfindungsgemäßen Verpackungseinheit auf die Haut, insbesondere auf die Haut der Achselhöhlen, aufgetragen wird/werden und für mindestens 1 Stunde, vorzugsweise für mindestens 2 Stunden, bevorzugt für mindestens 4 Stunden, insbesondere für mindestens 6 Stunden, auf der Haut der Achselhöhlen verbleibt/verbleiben.

Im Falle der erfindungsgemäßen Verpackungseinheit kann es vorgesehen sein, dass zunächst das kosmetische Mittel (M1) mit dem schweißhemmenden Wirkstoff und anschließend das kosmetisches Mittel (M2), enthaltend mindestens ein Chitosan der Formel (I), aufgetragen wird. Es ist jedoch auch möglich, zunächst das kosmetische Mittel (M2) aufzutragen und anschließend ein kosmetisches Mittel (M1) zu verwenden. Darüber hinaus können das kosmetische Mittel (M2) sowie das kosmetische Mittel (M1) auch gleichzeitig auf die Haut aufgetragen werden. Die Zeitspanne zwischen der Anwendung dieser beiden Mittel beträgt von 0 Sekunden bis 24 Stunden.

Weiterhin ist es im Rahmen dieses Erfindungsgegenstands bevorzugt, wenn das bzw. die kosmetischen Mittel nach dem Auftragen für mindestens 1 Stunde, vorzugsweise für mindestens 2 Stunden, bevorzugt für mindestens 4 Stunden, insbesondere für mindestens 6 Stunden, auf der Haut der Achselhöhlen verbleibt/verbleiben.

Bezüglich weiterer bevorzugter Ausführungsformen des erfindungsgemäßen Verfahrens gilt *mutatis mutandis* das zu den erfindungsgemäßen schweißhemmenden kosmetischen Mitteln sowie zu der erfindungsgemäßen Verwendung Gesagte.

Die vorliegende Erfindung wird insbesondere durch nachfolgende Punkte skizziert:
1. Schweißhemmendes kosmetisches Mittel, enthaltend
   a) mindestens einen Stoff, ausgewählt aus der Gruppe von kosmetischen Ölen, welche bei 20 °C und 1.013 hPa flüssig sind, Riechstoffen und Wachsen,
   b) Treibmittel in einer Gesamtmenge von 0 bis 99 Gew.-%, bezogen auf das Gesamtgewicht des schweißhemmenden kosmetischen Mittels, und
   c) mindestens ein Chitosan mit einer Viskosität von 15 bis 15.000 mPa*s, wobei das Chitosan die Formel (I) aufweist 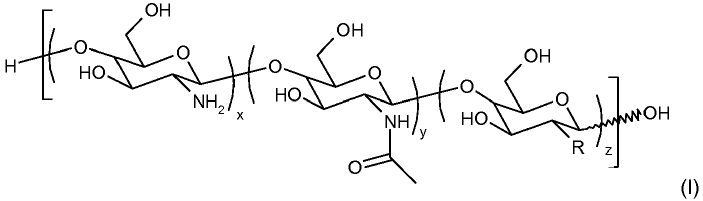 worin
      x und z, jeweils unabhängig voneinander, für ganze Zahlen von 5 bis 25.000 stehen,
      y für ganze Zahlen von 1 bis 25.000 steht,
      R für eine NH₂-Gruppe oder *-NH₃⁺CH₃CH(OH)C(O)O⁻ oder *-NH₃⁺OHCH₂C(O)O⁻ steht,
      mit der Maßgabe, dass die Reihenfolge der in der eckigen Klammer enthaltenden Einheiten mit den Indices x, y und z beliebig ist und dass der Rest R über * gebunden ist, und
   d) keine aluminiumhaltigen Verbindungen.
2. Schweißhemmendes kosmetisches Mittel nach Punkt 1, dadurch gekennzeichnet, dass die Viskosität mittels Brookfield RVDV II+, Spindel Nr. 2, 20 rpm, 20 °C unter Verwendung von 1 Gew.-% Chitosan der Formel (I) in einer 1 Gew.-%igen Essigsäurelösung, jeweils bezogen auf das Gesamtgewicht der Lösung, bestimmt wird.
3. Schweißhemmendes kosmetisches Mittel nach einem der Punkt 1 oder 2, dadurch gekennzeichnet, dass in der Formel (I) y für ganze Zahlen von 1 bis 22.000, vorzugsweise von 1 bis 20.000, bevorzugt von 1 bis 19.000, weiter bevorzugt von 1 bis 18.000, insbesondere von 1 bis 17.500, steht.
4. Schweißhemmendes kosmetisches Mittel nach einem der vorangehenden Punkte, dadurch gekennzeichnet, dass in der Formel (I) x und z, jeweils unabhängig voneinander, für ganze Zahlen von 5 bis 20.000, vorzugsweise von 6 bis 15.200, bevorzugt von 7 bis 13.000, weiter bevorzugt von 8 bis 12.500, insbesondere von 10 bis 11.700, stehen.
5. Schweißhemmendes kosmetisches Mittel nach einem der vorangehenden Punkte, dadurch gekennzeichnet, dass das mindestens eine Chitosan der Formel (I) einen Deacetylierungsgrad von 70 bis 99 %, vorzugsweise von 80 bis 98 %, bevorzugt von 80 bis 95 %, weiter bevorzugt von 80 bis 92 %, insbesondere von 80 bis 90 %, aufweist.
6. Schweißhemmendes kosmetisches Mittel nach einem der vorangehenden Punkte, dadurch gekennzeichnet, dass das mindestens eine Chitosan der Formel (I) aus maritimen Quellen, insbesondere Krabben, Garnelen, Krill, Pilzen, Zooplankton, Insekten, Mikroorganismen, modifizierte Mikroorganismen oder pflanzlichen Quellen isoliert ist.
7. Schweißhemmendes kosmetisches Mittel nach einem der vorangehenden Punkte, dadurch gekennzeichnet, dass das mindestens eine Chitosan der Formel (I) ein mittleres Molekulargewicht M_{w} von 5.000 bis 6.000.000 Da, vorzugsweise von 6.000 bis 5.500.000 Da, bevorzugt von 8.000 bis 5.050.000 Da, insbesondere von 10.000 bis 5.000.000 Da, aufweist.
8. Schweißhemmendes kosmetisches Mittel nach einem der vorangehenden Punkte, dadurch gekennzeichnet, dass das mindestens eine Chitosan der Formel (I) eine Viskosität von 15 bis 10.000 mPa*s, vorzugsweise von 15 bis 8.000 mPa*s, bevorzugt von 15 bis 6.000 mPa*s, insbesondere von 15 bis 5.000 mPa*s, aufweist, wobei die Viskosität jeweils mit Brookfield RVDV II+, Spindel Nr. 2, 20 rpm, 20 °C bestimmt wird und wobei zur Bestimmung der Viskosität 1 Gew.-% Chitosan der Formel (I) in einer 1 Gew.-%igen Essigsäurelösung, jeweils bezogen auf das Gesamtgewicht der Lösung, verwendet wird.
9. Schweißhemmendes kosmetisches Mittel nach einem der vorangehenden Punkte, dadurch gekennzeichnet, dass das mindestens eine Chitosan der Formel (I) in einer Gesamtmenge von 0,05 bis 40 Gew.-%, vorzugsweise von 0,1 bis 35 Gew.-%, bevorzugt von 0,2 bis 30 Gew-%, weiter bevorzugt von 0,4 bis 25 Gew.-%, insbesondere von 0,5 bis 20 Gew.-%, bezogen auf das Gesamtgewicht des treibmittelfreien schweißhemmenden kosmetischen Mittels, enthalten ist.
10. Schweißhemmendes kosmetisches Mittel nach einem der vorangehenden Ansprüche, dadurch gekennzeichnet, dass das schweißhemmende kosmetische Mittel einen pH-Wert von pH 2 bis pH 6 aufweist.
11. Verpackungseinheit (Kit-of-Parts), umfassend - getrennt voneinander konfektioniert -
   a) mindestens einen ersten Container (C1), enthaltend ein kosmetisches Mittel (M1) umfassend mindestens einen schweißhemmenden Wirkstoff, und
   b) mindestens einen zweiten Container (C2), enthaltend ein kosmetisches Mittel (M2) umfassend mindestens ein Chitosan der Formel (I) 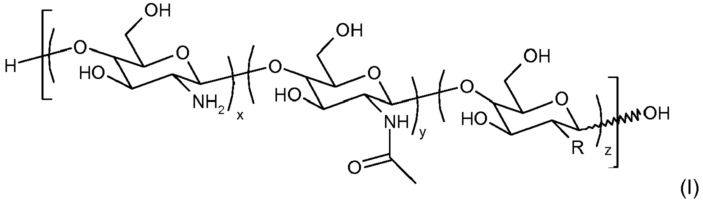 worin
      x und z, jeweils unabhängig voneinander, für ganze Zahlen von 5 bis 25.000 stehen,
      y für ganze Zahlen von 1 bis 25.000 steht,
      R für eine NH₂-Gruppe oder *-NH₃⁺CH₃CH(OH)C(O)O⁻ oder *-NH₃⁺OHCH₂C(O)O⁻ steht,
      mit der Maßgabe, dass die Reihenfolge der in der eckigen Klammer enthaltenden Einheiten mit den Indices x, y und z beliebig ist und dass der Rest R über * gebunden ist, und wobei das kosmetische Mittel (M2) keine aluminiumhaltigen Verbindungen enthält.
12. Verwendung mindestens eines Chitosans zur zumindest teilweisen Beeinflussung der Schweißdrüse(n), wobei das Chitosan die Formel (I) aufweist, 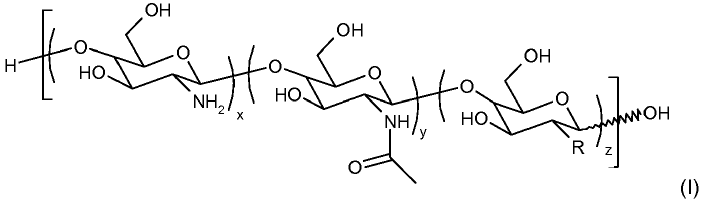 worin
   x und z, jeweils unabhängig voneinander, für ganze Zahlen von 5 bis 25.000 stehen,
   y für ganze Zahlen von 1 bis 25.000 steht,
   R für eine NH₂-Gruppe oder *-NH₃⁺CH₃CH(OH)C(O)O⁻ oder *-NH₃⁺OHCH₂C(O)O⁻ steht,
   mit der Maßgabe, dass die Reihenfolge der in der eckigen Klammer enthaltenden Einheiten mit den Indices x, y und z beliebig ist und dass der Rest R über * gebunden ist.
13. Verwendung einer Kombination, enthaltend
   a) mindestens einen Stoff, ausgewählt aus der Gruppe von kosmetischen Ölen, welche bei 20 °C und 1.013 hPa flüssig sind, Riechstoffen und Wachsen,
   b) Treibmittel in einer Gesamtmenge von 0 bis 99 Gew.-%, bezogen auf das Gesamtgewicht des schweißhemmenden kosmetischen Mittels, und
   c) mindestens ein Chitosan mit einer Viskosität von 15 bis 15.000 mPa*s, wobei das Chitosan die Formel (I) aufweist 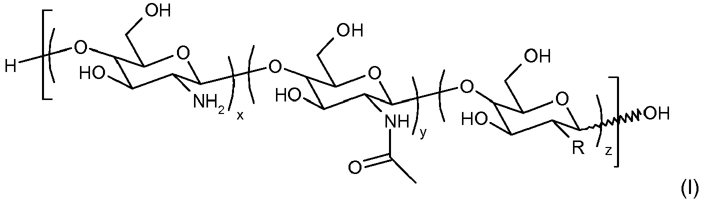 worin
      x und z, jeweils unabhängig voneinander, für ganze Zahlen von 5 bis 25.000 stehen,
      y für ganze Zahlen von 1 bis 25.000 steht,
      R für eine NH₂-Gruppe oder *-NH₃⁺CH₃CH(OH)C(O)O⁻ oder *-NH₃⁺OHCH₂C(O)O⁻ steht,
      mit der Maßgabe, dass die Reihenfolge der in der eckigen Klammer enthaltenden Einheiten mit den Indices x, y und z beliebig ist und dass der Rest R über * gebunden ist, und
   d) keine aluminiumhaltigen Verbindungen,
      zur Reduzierung und/oder Verhinderung von Schweiß, insbesondere Achselschweiß oder Schweiß anderer Körperregionen.
14. Nicht-therapeutisches kosmetisches Verfahren zur Verhinderung und/oder Reduzierung der Transpiration des Körpers, bei welchem ein schweißhemmendes kosmetisches Mittel nach einem der Punkte 1 bis 10 oder die kosmetischen Mittel (M1) und (M2) der Verpackungseinheit nach Punkt 11 auf die Haut, insbesondere auf die Haut der Achselhöhlen, aufgetragen wird/werden und für mindestens 1 Stunde, vorzugsweise für mindestens 2 Stunden, bevorzugt für mindestens 4 Stunden, insbesondere für mindestens 6 Stunden, auf der Haut der Achselhöhlen verbleibt/verbleiben.

Die folgenden Beispiele erläutern die vorliegende Erfindung, ohne sie jedoch darauf einzuschränken:

### Beispiele:

### 1. Bestimmung der Trübung

Die Bestimmung der Trübung, welche durch die folgenden Chitosane in einem pH-Bereich von 5,5 bis 7,5 bei einer pH-Wert Änderung von 0,5 bewirkt wird, wird folgendermaßen durchgeführt:

**Tabelle 1: Probenlösung (Angaben in Gew.-%)**

| | E-I* | E-II* |
|---|---|---|
| Chitosan 1 ^{a)} | 0,25 | -- |
| Chitosan 2 ^{b)} | -- | 0,10 |
| Milchsäure | 0,07 | 0,18 |
| HCl | Add pH | Add pH |
| Wasser | Add 100 | Add 100 |

| | | |
|---|---|---|
| * erfindungsgemäß ^{a)} R = *-NH₃⁺CH₃CH(OH)C(O)O⁻, M_{w} = 50.000 bis 1.000.000 Da, Viskosität = 70 bis 150 mPa*s, Deacetylierungsgrad ≥ 80 %, maritime Quelle ^{b)} R = *-NH₃⁺CH₃CH(OH)C(O)O⁻, M_{w} = 10.000 bis 20.000 Da, Viskosität = 20 bis 40 mPa*s, Deacetylierungsgrad ≥ 80 %, pflanzliche Quelle | | |

Für die Bestimmung der Trübung wurde ein Methrom Titrando 905 der Firma Methrom (USA) verwendet, welches mit einer Methrom Optrode 6.1115.000 sowie einer pH-Elektrode von Methrom ausgestattet ist. Die Steuerung des Methrom Titrando 905 erfolgt über die Software Tiamo der Firma Methrom. Zunächst wurden 30 ml einer Probenlösung gemäß Tabelle 1, welche einen pH-Wert von 3,0 aufwies, in dem offenen Probengefäß des Methrom Titrando 905 vorgelegt. Anschließend wurde bei 23 °C und 1.013 mbar unter Rühren (Rührgeschwindigkeit 8 des Titrando 905) solange kontinuierlich eine 1 Gew.-%-ige Natriumhydrogencarbonatlösung zugegeben, bis ein pH-Wert von 7,5 erreicht wurde. Während der Zugabe der 1 Gew.-%-igen Natriumhydrogencarbonatlösung wurde die Lichttransmission eines Lichtstrahls durch diese Probenlösung mit einer Methrom Optrode 6.1115.000 bei einer Wellenlänge von 574 nm (grüngelb) in mV (Auflösung 0,1 mV) gemessen. Jede Messung wird jeweils zweimal durchgeführt und hieraus der Mittelwert gebildet.

Die Trübung bzw. Veränderung der Lichtabsorption, welche durch die oben genannten Chitosane bewirkt wurde, wurde gemäß der Formel ΔL = [(|Lᵢ|/|L₀|]*100 bestimmt. In dieser Formel steht Lᵢ für die Differenz der Lichttransmission vor und nach Veränderung des pH-Wertes um mindestens 0,5 in dem pH-Bereich von 4,0 bis 8,0, bevorzugt von pH 4,5 und 7,5, insbesondere von pH 5,0 und 7,0. L₀ steht in dieser Formel für die Differenz aus der Lichttransmission bei pH 4,0 und bei pH 8,0, bevorzugt bei pH 4,5 und bei pH 7,5, insbesondere bei pH 5,0 und bei pH 7,0.

Bei einer pH-Wert Änderung von 0,5 zwischen pH 6,0 und pH 6,5 (Differenz Lichtabsorption bei pH 6,5 minus Lichtabsorption bei pH 6,0 bildet Wert Lᵢ) in einem pH-Wertbereich von 5,5 bis 7,5 (Differenz Lichtabsorption bei pH 7,5 minus pH 5,5 bildet Wert L₀) bewirkten diese Chitosane die in Tabelle 2 angegebene Veränderung der Lichtabsorption ΔL bzw. Trübung.

**Tabelle 2: Veränderung der Lichtabsorption ΔL bzw. Trübung**

| Probenlösung | ΔL [%] |
|---|---|
| E-I (Chitosan 1) | 30 |
| E-II (Chitosan 2) | 4 |

### 2. Formulierungen:

In den nachfolgenden Beispielen werden bevorzugt folgende Chitosane der Formel (I) eingesetzt:
- R = *-NH₃⁺CH₃CH(OH)C(O)O⁻, M_{w} = 50.000 bis 1.000.000 Da, Viskosität = 70 bis 150 mPa*s, Deacetylierungsgrad ≥ 80 %, maritime Quelle
- R = *-NH₃⁺CH₃CH(OH)C(O)O⁻, M_{w} = 50.000 bis 1.000.000 Da, Viskosität = 1.000 bis 5.000 mPa*s, Deacetylierungsgrad ≥ 80 %, maritime Quelle
- R = *-NH₃⁺OHCH₂C(O)O⁻, M_{w} = 500.000 bis 5.000.000 Da, Viskosität = 500 bis 5.000 mPa*s, Deacetylierungsgrad ≥ 80 %, maritime Quelle
- R = NH₂-Gruppe, M_{w} = 300.000 bis 2.000.000 Da, Viskosität = 150 bis 1.000 mPa*s, Deacetylierungsgrad ≥ 80 %, maritime Quelle
- R = NH₂-Gruppe, M_{w} = 50.000 bis 5.000.000 Da, Viskosität = 1.850 bis 2.250 mPa*s, Deacetylierungsgrad ≥ 80 %, maritime Quelle
- R = *-NH₃⁺CH₃CH(OH)C(O)O⁻, M_{w} = 10.000 bis 20.000 Da, Viskosität = 20 bis 40 mPa*s, Deacetylierungsgrad ≥ 80 %, pflanzliche Quelle

Erfindungsgemäße schweißhemmende kosmetische Mittel mit einem pH von 2,5 bis 6,0 (Mengenangaben in Gew.-%)

| | 1 | 2 | 3 | 4 | 5 | 6 | 7 |
|---|---|---|---|---|---|---|---|
| Isopropylmyristat | 0,50 | 0,10 | 0,50 | 1,0 | 2,0 | 3,0 | 5,0 |
| Chitosan Formel (I) | 0,05 | 0,1 | 0,2 | 0,3 | 0,4 | 1,5 | 2,0 |
| Eumulgin B3 ^{a)} | 3,0 | 3,0 | 3,0 | 4,0 | 4,0 | 4,0 | 5,0 |
| Parfum | 0,10 | 0,20 | 0,30 | 0,30 | 0,30 | 0,30 | 0,30 |
| Konservierungsmittel | 0,50 | 0,50 | 0,50 | 0,80 | 0,80 | 1,5 | 2,0 |
| pH-Stellmittel | ad pH | ad pH | ad pH | ad pH | ad pH | ad pH | ad pH |
| Wasser | ad 100 | ad 100 | ad 100 | ad 100 | ad 100 | ad 100 | ad 100 |

| | | | | | | | |
|---|---|---|---|---|---|---|---|
| ^{a)} Eumulgin B3 (INCI: Ceteareth-30; BASF) | | | | | | | |

Erfindungsgemäße schweißhemmende kosmetische Mittel mit einem pH von 2,5 bis 6,0 in Form von sprühbaren Emulsionen (Mengenangaben in Gew.-%)

| | 1 | 2 | 3 | 4 |
|---|---|---|---|---|
| Cyclopentasiloxan | 11 | 11 | 13 | 15 |
| Isopropylmyristat | 5,0 | 5,0 | 5,0 | 5,0 |
| Dow Corning ES-5227 DM ^{b)} | 6,0 | 6,0 | 7,0 | 7,0 |
| Parfum | 4,0 | 4,0 | 5,0 | 4,0 |
| Propylenglycol | 20 | 20,5 | 18 | 15 |
| Phenoxyethanol | 0,5 | 0,5 | 0,5 | 0,5 |
| Natriumbenzoat | 0,4 | 0,4 | 0,4 | 0,4 |
| Chitosan Formel (I) | 1,0 | 0,5 | 2,0 | 0,5 |
| Salzsäure, 20%ig | ad pH | ad pH | ad pH | ad pH |
| Wasser | ad 100 | ad 100 | ad 100 | ad 100 |

| | | | | |
|---|---|---|---|---|
| ^{b)} Dow Corning ES-5227 DM (INCI: Dimethicone, PEG/PPG-18/18 Dimethicone; Dow Corning) | | | | |

Erfindungsgemäße schweißhemmende kosmetische Mittel mit einem pH von 2,5 bis 6,0 in Form Rollons (Mengenangaben in Gew.-%)

| | 1 | 2 | 3 | 4 |
|---|---|---|---|---|
| Steareth-21 | 1,5 | 1,5 | 2,0 | 2,0 |
| Steareth-2 | 2,5 | 2,5 | 3,0 | 2,0 |
| PPG-15 Stearylether | 0,5 | 0,5 | 1,0 | 0,5 |
| Parfum | 1,0 | 1,0 | 1,5 | 1,0 |
| Tetranatrium EDTA | 0,1 | 0,1 | 0,1 | 0,1 |
| Chitosan Formel (I) | 1,0 | 0,5 | 2,0 | 0,5 |
| Salzsäure, 20%ig | ad pH | ad pH | ad pH | ad pH |
| Wasser | ad 100 | ad 100 | ad 100 | ad 100 |

## Patentansprüche

1. Verwendung mindestens eines Chitosans zur Vermeidung und/oder Verminderung der Absonderung von Schweiß aus dem Ausführungsgang von Schweißdrüsen, wobei das Chitosan die Formel (I) aufweist, 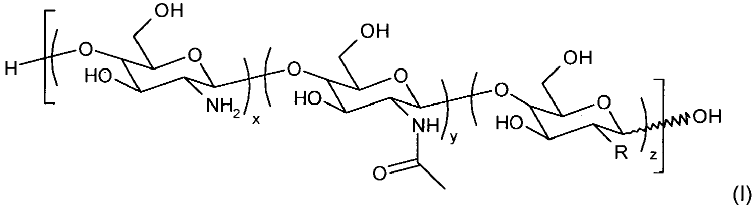 worin
x und z, jeweils unabhängig voneinander, für ganze Zahlen von 5 bis 25.000 stehen,
y für ganze Zahlen von 1 bis 25.000 steht,
R für eine NH₂-Gruppe oder *-NH₃⁺CH₃CH(OH)C(O)O⁻ oder *-NH₃⁺OHCH₂C(O)O⁻ steht,
mit der Maßgabe, dass die Reihenfolge der in der eckigen Klammer enthaltenden Einheiten mit den Indices x, y und z beliebig ist und dass der Rest R über * gebunden ist.

2. Verwendung nach Anspruch 1, **dadurch gekennzeichnet, dass** in der Formel (I) y für ganze Zahlen von 1 bis 22.000, vorzugsweise von 1 bis 20.000, bevorzugt von 1 bis 19.000, weiter bevorzugt von 1 bis 18.000, insbesondere von 1 bis 17.500, steht.

3. Verwendung nach einem der Ansprüche 1 oder 2, **dadurch gekennzeichnet, dass** in der Formel (I) x und z, jeweils unabhängig voneinander, für ganze Zahlen von 5 bis 20.000, vorzugsweise von 6 bis 15.200, bevorzugt von 7 bis 13.000, weiter bevorzugt von 8 bis 12.500, insbesondere von 10 bis 11.700, stehen.

4. Verwendung nach einem der vorangehenden Ansprüche, **dadurch gekennzeichnet, dass** das mindestens eine Chitosan der Formel (I) einen Deacetylierungsgrad von 70 bis 99 %, vorzugsweise von 80 bis 98 %, bevorzugt von 80 bis 95 %, weiter bevorzugt von 80 bis 92 %, insbesondere von 80 bis 90 %, aufweist.

5. Verwendung nach einem der vorangehenden Ansprüche, **dadurch gekennzeichnet, dass** das mindestens eine Chitosan der Formel (I) eine Viskosität von 15 bis 10.000 mPa*s, vorzugsweise von 15 bis 8.000 mPa*s, bevorzugt von 15 bis 6.000 mPa*s, insbesondere von 15 bis 5.000 mPa*s, aufweist, wobei die Viskosität jeweils mit Brookfield RVDV II+, Spindel Nr. 2, 20 rpm, 20 °C bestimmt wird und wobei zur Bestimmung der Viskosität 1 Gew.-% Chitosan der Formel (I) in einer 1 Gew.-%igen Essigsäurelösung, jeweils bezogen auf das Gesamtgewicht der Lösung, verwendet wird.

6. Verwendung nach einem der vorangehenden Ansprüche, **dadurch gekennzeichnet, dass** das mindestens eine Chitosan der Formel (I) in einer Gesamtmenge von 0,05 bis 40 Gew.-%, vorzugsweise von 0,1 bis 35 Gew.-%, bevorzugt von 0,2 bis 30 Gew-%, weiter bevorzugt von 0,4 bis 25 Gew.-%, insbesondere von 0,5 bis 20 Gew.-%, bezogen auf das Gesamtgewicht des treibmittelfreien schweißhemmenden kosmetischen Mittels, verwendet wird.

## Claims

1. The use of at least one chitosan for preventing and/or reducing the secretion of sweat from the excretory duct of perspiratory glands, wherein the chitosan comprises the formula (I), 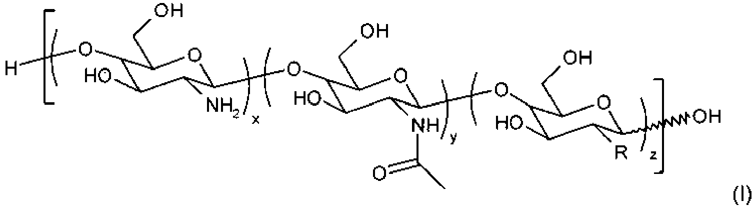 in which
x and z each represent, independently of one another, an integer of from 5 to 25,000,
y represents an integer of from 1 to 25,000,
R represents an NH2 group or *-NH₃⁺CH₃CH(OH)C(O)O⁻ or *-NH₃⁺OHCH₂C(O)O⁻, with the proviso that the sequence of the units contained in the square bracket having the indices x, y and z can be freely chosen and that the functional group R is bonded by means of *.

2. The use according to claim 1, **characterized in that** in formula (I), y represents an integer of from 1 to 22,000, preferably of from 1 to 20,000, more preferably of from 1 to 19,000, even more preferably of from 1 to 18,000, in particular of from 1 to 17,500.

3. The use according to one of claims 1 or 2, **characterized in that** in formula (I), x and z each represent, independently of one another, an integer of from 5 to 20,000, preferably of from 6 to 15,200, more preferably of from 7 to 13,000, even more preferably of from 8 to 12,500, in particular of from 10 to 11,700.

4. The use according to one of the preceding claims, **characterized in that** the at least one chitosan of formula (I) has a degree of deacetylation of from 70 to 99%, preferably of from 80 to 98%, more preferably of from 80 to 95%, even more preferably of from 80 to 92%, in particular of from 80 to 90%.

5. The use according to one of the preceding claims, **characterized in that** the at least one chitosan of formula (I) has a viscosity of from 15 to 10,000 mPa*s, preferably of from 15 to 8,000 mPa*s, more preferably of from 15 to 6,000 mPa*s, in particular of from 15 to 5,000 mPa*s, the viscosity being determined in each case using Brookfield RVDV II+, spindle no. 2, 20 rpm, 20 °C and, in order to determine the viscosity, 1 wt.% chitosan of formula (I) being used in 1 wt.% acetic acid solution, based in each case on the total weight of the solution.

6. The use according to one of the preceding claims, **characterized in that** the at least one chitosan of formula (I) is used in a total amount of from 0.05 to 40 wt.%, preferably of from 0.1 to 35 wt.%, more preferably of from 0.2 to 30 wt.%, even more preferably of from 0.4 to 25 wt.%, in particular of from 0.5 to 20 wt.%, based on the total weight of the propellant-free antiperspirant cosmetic agent.

## Revendications

1. Utilisation d'au moins un chitosane pour inhiber et/ou réduire excrétion sudorale par le canal excréteur des glandes sudoripares, dans laquelle le chitosane présente la formule (I), 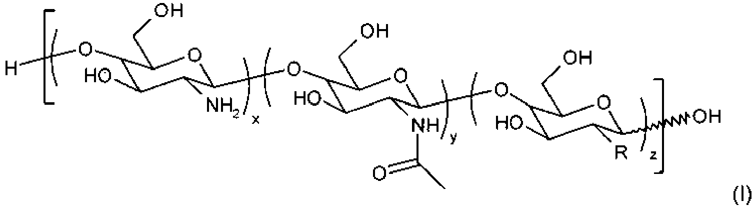 dans laquelle
x et z, chacun indépendamment l'un de l'autre, représentent des nombres entiers compris entre 5 et 25 000,
y représente des nombres entiers compris entre 1 et 25 000,
R représente un groupe NH₂ ou *-NH₃⁺CH₃CH(OH)C(O)O⁻ ou *-NH₃⁺OHCH₂C(O)O⁻, à condition que l'ordre des unités contenues dans des crochets avec les indices x, y et z soit quelconque et que le radical R soit lié par *.

2. Utilisation selon la revendication 1, **caractérisée en ce que** dans la formule (I), y représente des nombres entiers compris entre 1 et 22 000, de préférence entre 1 et 20 000, de préférence entre 1 et 19 000, de manière davantage préférée entre 1 et 18 000 et en particulier entre 1 et 17 500.

3. Utilisation selon l'une des revendications 1 ou 2, **caractérisée en ce que** dans la formule (I), x et z représentent, chacun indépendamment l'un de l'autre, des nombres entiers compris entre 5 et 20 000, de préférence entre 6 et 15 200, de préférence entre 7 et 13 000, de manière davantage préférée entre 8 et 12 500 et en particulier entre 10 et 11 700.

4. Utilisation selon l'une des revendications précédentes, **caractérisée en ce que** l'au moins un chitosane de formule (I) présente un degré de désacétylation compris entre 70 et 99 %, de préférence entre 80 et 98 %, de préférence entre 80 et 95 %, de manière davantage préférée entre 80 et 92 % et en particulier entre 80 et 90 %.

5. Utilisation selon l'une des revendications précédentes, **caractérisée en ce que** le au moins un chitosane de formule (I) présente une viscosité comprise entre 15 et 10 000 mPa*s, de préférence entre 15 et 8 000 mPa*s, de préférence entre 15 et 6 000 mPa*s, en particulier de 15 à 5 000 mPa*s, la viscosité étant déterminée avec Brookfield RVDV II+, broche n° 2, 20 tr/min, 20° C et 1 % en poids de la viscosité étant utilisé pour déterminer la viscosité dans une solution d'acide acétique à 1 % en poids de chitosane de formule (I), dans chaque cas par rapport au poids total de la solution.

6. Utilisation selon l'une des revendications précédentes, **caractérisée en ce que** l'on utilise l'au moins un chitosane de formule (I) en une quantité totale de 0,05 à 40 % en poids, de préférence de 0,1 à 35 % en poids, de préférence de 0,2 à 30 % en poids, de manière davantage préférée de 0,4 à 25 % en poids et en particulier de 0,5 à 20 % en poids, par rapport au poids total de l'agent cosmétique anti-transpirant exempt d'agent propulseur.
